(19) Europäisches Patentamt
European Patent Office
Office européen des brevets

(11) EP 3 012 257 A1

(12) **EUROPEAN PATENT APPLICATION**

(43) Date of publication:
**27.04.2016 Bulletin 2016/17**

(21) Application number: **14189765.2**

(22) Date of filing: **21.10.2014**

(51) Int Cl.:
*C07D 407/06* (2006.01) *A61K 31/366* (2006.01)
*A61P 35/02* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Studiengesellschaft Kohle mbH**
**45470 Mülheim (DE)**

(72) Inventors:
- **Fürstner, Alois**
**45478 Mülheim (DE)**
- **Mailhol, Damien**
**45470 Mülheim (DE)**
- **Willwacher, Jens**
**45470 Mülheim (DE)**

(54) **Leiodermatolide derivatives and their use**

(57) The present invention refers to leiodermatolide derivatives and the use thereof as cytotoxic agents, in particular in the form of ADC s.

Figur 1

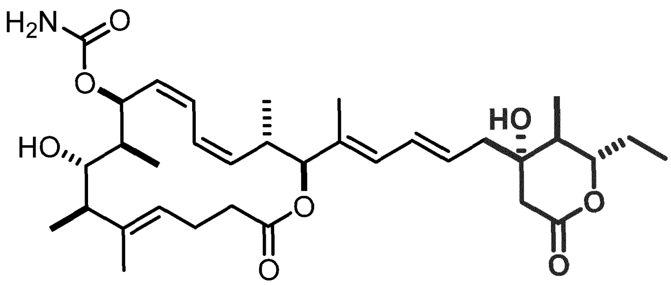

Leiodermatolide (**1**)

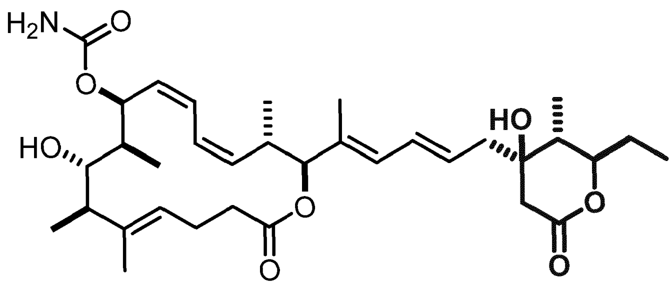

Leiodermatolide (2)

EP 3 012 257 A1

## Description

The present invention refers to leiodermatolide derivatives, their use as cytotoxic agents and as cytotoxic payloads of antibody-drug-conjugates (ADCs).

Of great importance to mankind is the control of pathological cellular proliferation such as that which occurs in the case of cancer. Considerable research and resources have been devoted to oncology and antitumor measures including chemotherapy. While certain methods and chemical compositions have been developed which aid in inhibiting, remitting, or controlling the growth of, for example, tumors, new methods and antitumor chemical compositions are needed. Anti-proliferative agents can also be useful in treating autoimmune diseases and inflammatory diseases.

Mankind has used natural products and natural product derivatives for a long time to treat various kinds of ailments, including cancer and autoimmune diseases. A prominent example is the diterpene commonly known as paclitaxel, which had originally been isolated from several species of yew trees. Paclitaxel is a mitotic spindle poison that stabilizes microtubules and inhibits their depolymerization to free tubulin. Paclitaxel itself (Taxol®) and derivatives thereof have been approved as drugs for clinical use; for a review on Paclitaxel and other anticancer agents from natural products, see: G. M. Cragg, D. G. I. Kingston, D. J. Newman (Eds.), Anticancer Agents from Natural Products, CRC Press, 2005.

Marine sponges have also proven to be rich sources of biologically active chemical molecules. A number of publications disclose organic compounds derived from marine sponges, including US8431724.

Amongst those sponges, a lithistid *Leiodermatium* sponge collected in deep waters off the Florida coastline can be mentioned. The bioassay-guided fractionation of the crude extracts thereof led to the isolation of leiodermatolide (1).

This macrolide is peculiar in that it was found to elicit abnormal mitotic spindle formation in two different cancer cell lines in the nM range although purified tubulin remained unaffected even at 20 μM concentration. These preliminary data have to be seen in the light of what is known about other anti-mitotic agents, which continue to represent the mainstay of systemic cancer chemotherapy. The clinically approved drugs of this functional type usually bind to tubulin as the primary target and, in doing so, disrupt the dynamic of microtubule assembly/disassembly in one way or the other. Paclitaxel mentioned above is a typical example for this mode of action. Leiodermatolide (1) seems to be distinct in its mode of action and therefore merits closer scrutiny, in particular as it exhibited impressive antiproliferative activity against a small panel of human and murine cancer cell lines.

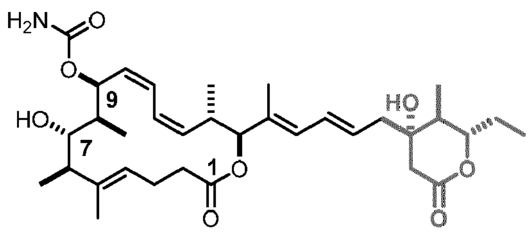

Leiodermatolide (**1**)

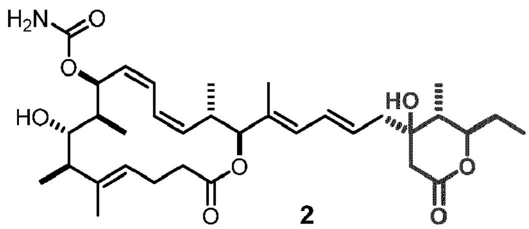

**2**

The PCT-patent application WO 2008/019135 discloses compounds of the formula:

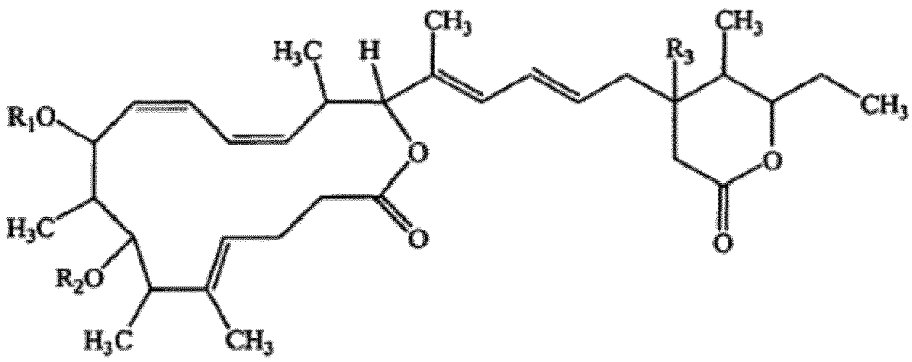

but mainly theoretically only. The inventors of said PCT-application were unable to fully identify the stereochemistry of the basic leiodermatolide ring and furthermore, not a single compound having said basic structure except the natural product leiodermatolide itself has been identified or prepared by them. Partial stereostructures of leiodermatolide were later disclosed in: Paterson, I.; Dalby, S. M.; Roberts, J. C.; Naylor, G. J.; Guzman, E. A.; Isbrucker, R.; Pitts, T. P.; Linley, P.; Divlianska, D.; Reed, J. K.; Wright, A. E. Angew. Chem. Int. Ed. 2011, 50, 3219-3223

The inventors here were recently able to fully clarify the stereochemistry of leiodermatolide *(*Angew. Chem. Int. Ed. 2012, 51, 12041-46). To this end, compound **(1)** and the diastereomer **(2)** were prepared, which are comprised of the same macrolide core but feature two antipodal δ-lactone segments (color coded). Although the NMR spectra of these two conceivable representations of leiodermatolide, recorded at 600 MHz, are almost indistinguishable, very subtle differences in a subsector of the aliphatic region of the $^1H$ NMR spectrum (2.20-2.50 ppm) allowed the inventors to assign the full stereostructure of leiodermatolide **(1)** as shown in Figure 1. The absolute configuration was inferred from the optical rotation data. A later independent synthesis by Paterson and coworkers *(*Angew. Chem. Int. Ed. 2014, 53, 2692-2995) confirmed the inventor's conclusions.

In their subsequent studies, the inventors now developed a strategy for modifying the leiodermatolide framework. This allows, inter alia, to link leiodermatolide **(1)** or a derivative thereof to a monoclonal antibody in order to provide an antibody-drug-conjugate (ADC).

Antibody-drug conjugates (ADCs) use monoclonal antibodies to deliver a potent cytotoxic compound selectively to tumor cells, thus improving the therapeutic index of a given chemotherapeutic agent serving as the cytotoxic payload. Traditional cancer chemotherapy is often accompanied by systemic toxicity to the patient. On the other hand, monoclonal antibodies against antigens on cancer cells offer an alternative tumor-selective treatment approach, but most monoclonal antibodies themselves are not sufficiently potent to be therapeutically active on their own. For a review on the state of the art of ADCs, see: R. V. J. Chari, M. L. Miller, W. C. Widdison, Angew. Chem. Int. Ed. 2014, 53, 3796.

Generally speaking, clinically viable ADCs consist of a cytotoxic payload connected to a monoclonal antibody by an appropriate linker. Antibodies are large proteins. Their amino acid residues possess free carboxy groups, free hydroxyl groups, free amino groups, and/or free thiol groups that can be used to ligate the antibody to a cytotoxic payload via a suitable linker. The linker between the antibody and the payload may or may not be cleaved upon internalization of the antibody-drug-conjugate into the targeted cell to release the active drug compound. Premature cleavage and hence premature release of the drug compound in circulation in a patient, however, can lead to systemic toxicity and a lower therapeutic index. Therefore the design of the linker has to balance the need of good stability during circulation and efficient cleavage upon delivery to the targeted cancer cell. This balance needs to be defined in preclinical and clinical studies and cannot be generalized. It also depends on the type of cancer to be treated and on the nature of the payload. For this reason, the present invention cannot be limited to any particular type of clinically viable linker nor to any particular way in which the linker is connected via a hemistable bond to the chosen antibody. The literature shows that preferred (hemistable) connections between an appropriate antibody and the linker are bonds of the following types: amide, ester, hydrazone, acyl hydrazone, oxime, thioether, disulfide, carbamate, carbonate. A suitable linker must therefore contain at least one functional group which is capable of forming one of said bonds upon reaction with a monoclonal antibody (R. V. J. Chari, M. L. Miller, W. C. Widdison, Angew. Chem. Int. Ed. 2014, 53, 3796).

The present invention deals with the problem how to modify the basic ring structure of leiodermatolide **(1)** so that leiodermatolide **(1)** or analogues can be used as cytotoxic payload for the preparation of an ADC while maintaining the cytotoxic activity. In more detail, the present invention could identify the proper sites at which a linker can be attached without severely compromising or even annihilating the cytotoxic activity of leiodermatolide **(1)** or an analogue thereof. The literature demonstrates that conjugation of a cytotoxic compound to a monoclonal antibody can lead to significantly decreased potency compared to the parent free drug, which can thwart the clinical success (review: R. V. J. Chari, M. L. Miller, W. C. Widdison, Angew. Chem. Int. Ed. 2014, 53, 3796). Therefore, the site of attachment of the linker to the cytotoxic payload is of critical importance.

No information whatsoever about the pharmacophore of leiodermatolide (1) had been available when the invention was made. Specifically, it was not clear if any site of leiodermatolide can be chemically modified without significantly compromising or even annihilating the cytotoxicity of this compound. Only as such a site could be identified by the present invention, leiodermatolide (1) or derivatives thereof can ultimately be used as a cytotoxic payload of an ADC.

To identify such viable sites, the inventors modified the substituents on and/or the substructures within the framework of leiodermatolide **(1)** and prepared a set of non-natural analogues. The cytotoxicity of these derivatives and analogues was compared to the cytotoxicity of leiodermatolide **(1)** itself. The analysis of these data showed which sites and substructures of the framework of this natural product are chemically viable for modification without losing or unduly compromising the biological activity of the natural lead compound. In this way, the inventors proved that leiodermatolide **(1)** qualifies as cytotoxic payload for antibody-drug-conjugates (ADCs). Moreover, the invention delineates the preferred linker sites which can be used for the preparation of antibody-drug-conjugates comprising leiodermatolide (**1**) or a derivative thereof as the cytotoxic payload.

Thus, the general inventive concept of the present invention refers to an antibody-drug-conjugate comprising a leiodermatolide derivative of formula (I) as the cytotoxic payload :

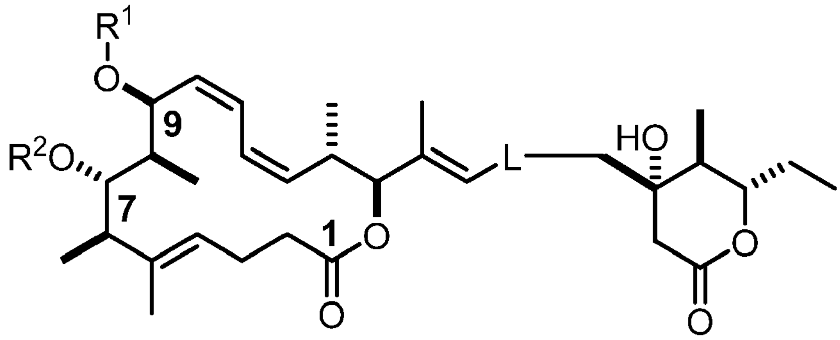

Formula (I)

connected to a monoclonal antibody by one of the clinically viable linker $R^1$ and $R^2$ which are connected to either the carbon atom C.7 or the carbon atom C.9 of said leiodermatolide derivative, wherein L represents a trans -CH=CH- group or a-$CH_2$-$CH_2$- group.

The present invention is therefore directed to a leiodermatolide derivative and its antibody conjugates as claimed in the claims. The compounds of WO2008/019135 are not subject matter of the present application and are excluded from the scope of the present invention.

In more detail, the present invention is directed to a leiodermatolide derivative of the formula (I)

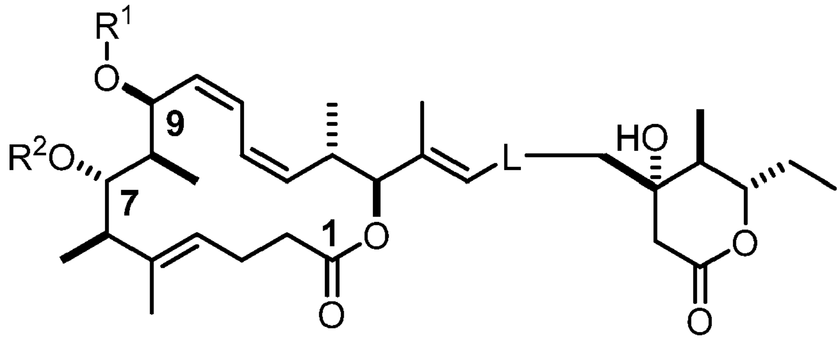

Formula (I)

wherein:

$R^1$ is -CO-$NR_2$, wherein R is the same or different and is hydrogen or a optionally substituted $C_1$ to $C_6$-alkyl group,

or $R^1$ is a linking group, which is capable of linking the leiodermatolide unit to an antibody;
$R^2$ is hydrogen, an optionally substituted $C_1$ to $C_6$-alkyl group or $R^2$ is a linking group, which is capable of linking the leiodermatolide unit to an antibody;
L represents a trans -CH=CH- group or a $-CH_2-CH_2-$ group,
with the proviso that

a. only one of $R^1$ and $R^2$ is a linking group, and
b. the compound is excluded wherein $R^1$ is $-CO-NH_2$, $R^2$ is hydrogen and L represents a trans -CH=CH- group.

Thus, the inventive leiodermatolide derivative of the formula (I) does not comprise the natural leiodermatolide, but a leiodermatolide of the formula (I) with $R^1$ or $R^2$ as defined before as well as the L=$-CH_2-CH_2-$ group containing derivative.

In said leiodermatolide derivative of the formula (I), $R^1$ or $R^2$ may preferably be a linking group having at least one functional group, said functional group being preferably terminal to the leiodermatolide unit and said functional group being capable of chemically bonding to an antibody. In view of steric requirements, only one of $R^1$ and $R^2$ should be a linker which is capable of chemically bonding the leiodermatolide unit to an antibody.

$R^1$ or $R^2$ may each be a linking group having preferably a hydrocarbon chain, optionally comprising one or more units such as alkylene groups, arylene groups, heteroarylene groups or different ("mixed") groups thereof in the chain, which chain may also contain one or more heteroelements such as oxygen, sulfur or nitrogen in the chain. The chain might thus comprise -O-, -S-, -NR-, sulfur-sulfur units (-SS-), carbonic or carboxylic acid derivative units such as -(CO)-(NH/O/S)- or its isomers such as esters, lactones, amides, carbamates, carbonates or hetero-analogs thereof such as -C(O)O-, -C(O)NR-, -O-C(O)NR-, -S-C(O)NR-, - OC(S)NR-, -S-C(S)NR-, R being hydrogen or a hydrocarbon group having 1 to 20, preferably 1 to 6 carbon atoms, in the chain between said alkylene units, arylene units, heteroarylene units or different ("mixed thereof") units. Thus for exemplary purposes, any of said alkylene group, arylene group or heteroarylene group might be bonded to each other either via a carbon atom or a heteroatom in the chain and might also include one or more polyoxyalkylene units, for example.

Said hydrocarbon chain of $R^1$ and $R^2$ may have 1 to 40, more preferably 6 to 30, even more preferably 6 to 20 carbon atoms in the chain , which chain may also contain one or more heteroelements such as oxygen, sulfur or nitrogen in the chain as defined before, and is preferably substituted terminally to the leiodermatolide unit with one or more substituents including heterosubstituents selected from =O, -OH, -SH, -SSR, -COOR, $-C(O)NH_2NH_2$, $-CO-NR_2$, $-NR_2$, ,-N(C(O)CH=CHC(O))-, $-OC(O)CH_2X$, -X, X being halogen, R being same or different and being hydrogen or a hydrocarbon group having 1 to 20, preferably 1 to 6 carbon atoms.

The present invention is also directed to a leiodermatolide derivative of the formula (I) wherein $R^1$ is $-(CO)_n-(NH/O/S)_m-$Rw wherein Rw is a linear or branched polyoxyalkylene hydrocarbon group or a linear or a branched polyalkylene hydrocarbon group, which hydrocarbon group may also contain one or more heteroelements such as oxygen, sulfur or nitrogen in the chain as defined before and may have preferably 1 to 20, more preferably 1 to 12 carbon atoms which may be preferably terminally substituted with one or more substituents selected from =O, -OH, -SH, -SSR, -COOR, $-C(O)NH_2NH_2$, $-CO-NR_2$, $-NR_2$, - N(C(O)CH=CHC(O))-, $-OC(O)CH_2X$, -X, X being halogen, R being same or different and being hydrogen or a hydrocarbon group having 1 to 20, preferably 1 to 6 carbon atoms,
$n = 0$ or 1; $m = 0$ or 1 with $m = 0$ when $n = 0$;
$R^2$ is hydrogen or a optionally substituted $C_1$ to $C_6$-alkyl group, and
L represents a trans -CH=CH- or $-CH_2-CH_2-$ group.

The present invention is furthermore directed a leiodermatolide derivative of the formula (I) wherein $R^1$ is $-CO-NR_2$, wherein R is the same or different and is hydrogen or a optionally substituted $C_1$ to $C_6$-alkyl group;
$R^2$ is $-(CO)_n-(NH/O)_m-$Rw wherein Rw is a linear or branched polyoxyalkylene hydrocarbon group or a linear or a branched polyalkylene hydrocarbon group, which hydrocarbon group may also contain one or more heteroelements such as oxygen, sulfur or nitrogen in the chain as defined before and may have preferably 1 to 20, more preferably 1 to 12 carbon atoms which group may be preferably terminally substituted with one or more substituents selected from from O, -OH, - SH, -SSR, -COOR, $-C(O)NH_2NH_2$, $-NR_2$, -N(C(O)CH=CHC(O))-, $-OC(O)CH_2X$, -X, X being halogen, R being hydrogen or a hydrocarbon group having 1 to 20, preferably 1 to 6 carbon atoms,
$n = 0$ or 1; $m = 0$ or 1 with $m = 0$ when $n = 0$, and
L represents a trans -CH=CH- or $-CH_2-CH_2-$ group.

The present invention also includes a leiodermatolide derivative of the formula (I) wherein:

$R^1$ is $-CO-NR_2$, wherein R is the same or different and is hydrogen or a optionally substituted $C_1$ to $C_6$-alkyl group;
$R^2$ is hydrogen, a optionally substituted $C_1$ to $C_6$-alkyl group;
L represents a $-CH_2-CH_2-$ group.

In more detail, any of the hydrocarbon groups may be linear or branched and may comprise indicated groups having the carbon number as indicated as long as the capability of the linking group to link the leiodermatolide unit to the antibody is not negatively influenced.

$C_1$-$C_{20}$-alkyl can be straight chain, branched or cyclic and may have 1, 2, 3, 4, 5, 6, 7, 8, 9, 10, 11, 12, 13, 14, 15, 16, 17, 18, 19 or 20 carbon atoms. Alkyl might be lower alkyl such as $C_1$-$C_6$-alkyl, in particular methyl, ethyl, propyl, isopropyl, butyl, isobutyl, sec-butyl or tert-butyl, likewise pentyl, 1-, 2- or 3-methylpropyl, 1,1-, 1,2- or 2,2-dimethylpropyl, 1-ethylpropyl, hexyl, 1-, 2, 3- or 4-methylpentyl, 1,1-, 1,2-, 1,3-, 2,2-, 2,3- or 3,3-dimethylbutyl, 1- or 2-ethylbutyl, 1-ethyl-1-methylpropyl, 1-ethyl-2-methylpropyl, 1,1,2- or 1,2,2-trimethylpropyl.

Cycloalkyl might be $C_3$-$C_{10}$-alkyl and may be cyclopropyl, cyclobutyl, cyclopentyl, cyclohexyl or cycloheptyl.

Halogen is F, Cl, Br or I.

Alkoxy might be $C_2$-$C_{10}$ alkoxy such as methoxy, ethoxy, propoxy, iso-propoxy, *tert*-butoxy etc.

Optionally substituted means unsubstituted or monosubstituted, disubstituted, trisubstituted, tetrasubstituted, pentasubstituted, or even further substituted foreach hydrogen on the hydrocarbon.

Including heteroatoms and/or aromatic hydrocarbons in the chain means that one or more carbon atoms in the chain might be replaced by heteroatoms such as N, O or S or be part of an aromatic ring structure.

Aryl might be phenyl, naphthyl, biphenyl, anthracenyl, and other polycondensed aromatic systems and might be aryl having an alkyl substituent such as $C_1$ to $C_6$ alkyl.

Aryl-($C_1$-$C_6$)-alkyl might be benzyl or substituted benzyl.

Heteroaryl having one or more heteroatoms selected from among N, O and S is preferably 2- or 3-furyl, 2- or 3-thienyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, also preferably 1,2,3-triazol-1-, -4- or -5-yl, 1,2,4-triazol-1-, -3- or -5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 3- or 4-pyridazinyl, pyrazinyl, 1-, 2-, 3-, 4-, 5-, 6- or 7-Indolyl, 4- or 5-isoindolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 2-, 3-, 4-, 5-, 6-, 7- or 8-quinolyl, 1-, 3-, 4-, 5-, 6-, 7- or 8-isoquinolyl, 3-, 4-, 5-, 6-, 7- or 8-cinnolinyl, 2-, 4-, 5-, 6-, 7- or 8-quinazolinyl, 5- or 6-quinoxalinyl, 2-, 3-, 5-, 6-, 7- or 8-2H-benzo-1,4-oxazinyl, also preferably 1,3-benzodioxol-5-yl, 1,4-benzodioxan-6-yl, 2,1,3-benzothiadiazol-4- or -5-yl or 2,1,3-benzoxadiazol-5-yl.

The present invention is also referred to in the attached Figures. Said Figures further illustrate the invention and show:

Figure 1: Leiodermatolide (1) and the non-natural diastereomer (2);

Figure 2: Novel Derivatives of Leiodermatolides (1) and (2);

Figure 3: Preparation of the Alcohol Segment with Reagents and Conditions as follows:

a) $CHI_3$, NaH, $Et_2O$, reflux, 99%; b) KOH, EtOH/$H_2O$, reflux, 91%; c) $BH_3$(THF), THF, -30°C → RT; d) [Cu(MeCN)$_4$]OTf (5 mol%), bipyridine (5 mol%), TEMPO (5 mol%), N-methylimidazole (10 mol%), MeCN, 55% (over both steps); e) **19,** $Cy_2BOTf$, $Et_3N$, $CH_2Cl_2$, -78°C → RT, 76%; f) TBSOTf, 2,6-dimethyl-pyridine, $CH_2Cl_2$, -10°C; g) DIBAI-H, toluene, -78°C, 83% (over both steps); h) Dess-Martin periodinane, $CH_2Cl_2$, 0°C, 96%; i) **20,** KHMDS, THF, -55°C, 51% (*Z:E* > 20:1) (over both steps); j) TBAF, THF, 0°C, 98%

Figure 4: Preparation of the Acid Segment with Reagents and Conditions as follows:

a) $Bu_2BOTf$, $Et_3N$, propanal, 98%; b) $SO_3$·pyridine, $CH_2Cl_2$, DMSO, $Et_3N$, -15°C, 75%; c) 25 (5 equiv.), Sn(OTf)$_2$, $Et_3N$, $CH_2Cl_2$, -20°C, 83% (dr = 9:1); d) $Me_4NBH(OAc)_3$, HOAc, MeCN, -50°C, 95% (dr = 92:8); e) TBSOTf, $Et_3N$, $CH_2Cl_2$, -78°C → 0°C, 89%; f) (MeO)NHMe·HCl, $AlMe_3$, THF, 0°C → RT, 92%; g) MOMCl, (*i*Pr)$_2$NEt, DMF, 50°C, 98%; h) MeMgCl, $Et_2O$, 0°C, 98%; i) $CH_2$=CHMgCl, THF,-78°C → RT, 92%; j) $PBr_3$, pyridine, $Et_2O$, 0°C, 83%; k) EtOAc, LDA, CuI, THF,-110°C → -30°C, 58%; l) $Me_3SiOK$, $Et_2O$, 98%

Figure 5: Preferred Synthesis of the δ-Lactone Segment with Reagents and Conditions as follows:
a) $Bu_2BOTf$ (2 equiv.), $Et_3N$, propanal, $Et_2O$, -78°C, 74% (dr = 11:1); b) $Ac_20$, $Et_3N$, DMAP cat. $CH_2Cl_2$, 0°C, 82%; c) LiHMDS, THF, -78°C, 83%; d) (1 *R*)-**12** (1.1 equiv.), 86% (dr = 5.5:1); e) see Table 3

Table 3. Catalytic asymmetric allylation or propargylation of ß-ketolactone **9.**

| Entry | Reagent | Catalyst | Yield | 8 (31): *epi* |
|---|---|---|---|---|
| 1 | **32** | R-**34** | 15-40%[b,c,d] | 4:1 |
| 2 | **32** | *R*-**34** | 84%[d] | 6.2:1 |

(continued)

| Table 3. Catalytic asymmetric allylation or propargylation of ß-ketolactone **9.** | | | | |
|---|---|---|---|---|
| Entry | Reagent | Catalyst | Yield | 8 (31): *epi* |
| 3 | **32** | *R*-**34** | 95% | 6.1:1 |
| 4 | **32** | *S*-**34** | 88%[d] | 1:9.0 |
| 5 | **33** | *R*-**34** | 94% | 7.6:1 |
| 6 | **33** | *R*-**34**[e] | 80% | 6.6:1 |
| 7 | **33** | *S*-**34** | 87% | 1:15 |
| [a] unless stated otherwise, all reactions were performed with **34** (10 mol%) in toluene at 130°C under microwave heating; [b] converions (GC); [c] at ambient temperature without microwave heating; [d] in the presence of *t*BuOH (2 equiv.); [e] using only 2 mol% of **34** | | | | |

Figure 6: Preparation of the Stannane Donor with Reagents and Conditions as follows:

a) $Bu_3SnH$ (over 15 min), $Pd_2(dba)_3$ (1 mol%), $PCy_3{\cdot}HBF_4$ (4 mol%), *i*$PrNEt_2$ (8 mol%), $CH_2Cl_2$, *E*-**35** (72%) and **36** (15%)

Figure 7: Completion of the total synthesis of leiodermatolide **(1)** with Reagents and Conditions as follows:

a) EDCl·HCl, DMAP, $CH_2Cl_2$, 0°C, 96%; b) **11** (40 mol%), $CH_2Cl_2$/toluene, 100°C, 72%; c) TBAF, THF, 4A MS, 0°C, 88%; d) **10** (15 mol%), toluene, RT, 61%; e) **35,** $Pd(PPh_3)_4$ (5 mol%), CuTC, $[Ph_2PO_2][NBu_4]$, DMF, 93% (from **3),** 90% (from **38);** f) TBAF, THF, 4A MS, 0°C, 81%; g) Zn(Cu/Ag), THF/$H_2O$/MeOH, 50°C, 92%; h) $Cl_3CC(O)NCO$, $CH_2Cl_2$, -78°C, then $Al_2O_3$, 75%; i) $Me_2BBr$, $CH_2Cl_2$, -90°C → 78°C, 86%

Figure 8: Synthesis of analogues **43** and **44,** and
Figure 9:. Synthesis of analogues **42, 45** and **46.**
Figure 3-7 show the optimized synthesis of leiodermatolide **(1).** Figures 3-9 also show the flexibility of the chosen synthesis route. If desirable, it allows functional groups other than the ones present in **1, 2, 42, 44, 45** or **46** to be attached to the C.7 and/or C.9 position of leiodermatolide (1) or analogues thereof. This includes, but is not limited to, other esters, ethers, acetals, carbamates, carbonates, or thiocarbonates.

The analogues and derivatives of leiodermatolide, which were used to identify the pharmacophore and hence the preferred linker sites for the preparation of ADCs were prepared analogously. Details are shown in Figures 8 and 9, although not all individual steps were optimized. These Figures also show additional derivatives of leiodermatolide **(1).**

The representative derivatives and analogues compiled in Figure 2 have been prepared by the inventors as shown in the experimental section below, although the specific synthesis steps have not been optimized. The compounds comprise structural point mutations as used by the inventors that served the following purposes as intended by the inventors:

(1) Compound **2** had originally been considered as the other possible candidate to represent leiodermatolide *(*Angew. Chem. Int. Ed. 2012, 51, 12041-46); it comprises the identical macrolide portion but differs from the natural product by the antipodal head group. While leiodermatolide **(1)** and the diastereomer **(2)** are hardly distinguishable by spectroscopic means, the comparison of their bioactivity can unravel a possible functional link between the segregated stereoclusters of the macrolactone on the one hand and the δ-lactone on the other hand.

(2) In compound **42,** one of the double bonds of the spacer has been edited out to reduce its stiffness.

(3) Compounds **43** and **44** comprise further changes in the head group of leiodermatolide **(1).** Because the δ-lactone might be hydrolysis-prone, this entity was replaced by an arguably more labile methyl ester on the one hand and by a cyclohexanol ring on the other hand which is not amenable to hydrolysis.

(4) The possibility that a hydrogen bond between the carbamate carbonyl attached to C.9 and the C.7-OH group is structure determining and therefore also functionally relevant was probed in the following ways: Compound **41** as the immediate precursor of leiodermatolide **(1)** but with a MOM-acetal in place was subjected to testing as a putative "negative" control. Conversely, compound **45** was prepared which features an acetate instead of the carbamate as an only gradually less potent hydrogen bond acceptor. Finally, product **46** with a six-membered cyclic acetal can be seen as a rigid covalent linker in lieu of the hydrogen bonding array in question.

**Cytotoxicity:** Leiodermatolide **(1)** showed excellent potencies in tumor cell proliferation assays using a select panel of seven human cancer cell lines. The $GI_{50}$'s after 4 days incubation were in the range of 0.4-3.5 nM (Table 1). Of particular note is the fact that this impressive potency was also observed for the HEL92.1.7 cell line that expresses the Pgp efflux transporter, which is a major cause of resistance of leukemias against cancer chemotherapy. This may be taken as an indication that leiodermatolide **(1)** does not constitute an effective efflux substrate.

Table 1. Effect of leiodermatolide **(1)** on cancer cell cytotoxicity after 4 days treatment; GI = growth inhibition

| Cell line | Histotype | $GI_{50}$ (nM) |
|---|---|---|
| N87 | gastric | 2.4 |
| MDA-MB-361-DYT2 | breast | 3.5 |
| HT29 | colon | 2.5 |
| HL60 | leukemia | 1.0 |
| NB4 | leukemia | 0.4 |
| HEL92.1.7 | leukemia | 1.0 |
| Raji | leukemia | 0.9 |

Next, the set of analogues and synthetic intermediates prepared by the inventors was assayed. $GI_{50}$ values were determined for those compounds for which a first dosing screen had indicated appreciable activity < 1 μM. In addition to leiodermatolide (1) itself, five compounds passed this threshold (Table 2). The most potent of these is product **45** bearing an acetate moiety instead of the carbamate at C.9, followed by the dihydro derivative **42** comprising a single olefin instead of the 1,3-diene within the side chain. Compound **41** as the MOM-protected derivative of leiodermatolide **(1)** is less potent for all but two of the tested cell lines. The rigidified compound **46** with a cyclic acetal on the backbone was less active and did not meet the 1 μM cut-off.

Moreover, the inventors have found that compound **2** comprising the antipodal δ-lactone is at least two orders of magnitude less potent than **1,** even though these two diastereomers are almost indistinguishable by NMR. This differential provides an unambiguous biological confirmation for the original structure assignment as provided by the inventors earlier on and reveals an intimate functional link between the two lactone subunits of **1,** albeit their interrelation is spectroscopically almost intangible. This comparison also shows that the specific stereochemistry of the δ-lactone is of critical importance.

Analogue **44** with the cyclohexyl head group retains sub-micromolar activity, whereas analogue **43** terminated by a simple methyl ester had already been sorted out during the prescreening. These findings suggest that the specific decoration as well as the stereochemistry of the δ-lactone in leiodermatolide **(1)** is functionally relevant; one possibility is that it imparts chemical stability.

**Table 2.** Assessment of the cytotoxicity ($GI_{50}$, nM) of analogues after 4 days treatment

| Compound | HL60 | NB4 | HEL | Raji | HT29 | N87 | MDA[a] |
|---|---|---|---|---|---|---|---|
| **1** | 1.0 | 0.4 | 1.0 | 0.9 | 2.5 | 2.4 | 3.5 |
| **45** | 13.0 | 17.5 | 1.4 | 67.9 | 5.9 | 8.0 | >10 |
| **42** | 35.8 | 14.8 | 51.0 | 66.5 | 40.6 | 41.1 | 52.6 |
| **41** | >100 | >100 | >100 | >100 | >100 | 50.4 | 44.6 |
| **2** | nd | nd | nd | nd | 199 | 144.9 | 306.5 |
| **44** | nd | nd | nd | nd | 480.2 | 288.6 | 422.3 |
| [a] MDA-MB-361-DYT2 cell line; nd = not determined | | | | | | | |

The inventors found that the synthetic congeners **45** and **42** of leiodermatolide **(1)** are exquisitely potent vis-à-vis a panel of human cancer cell lines, including a leukemia cell line expressing the Pgp efflux transporter. Additional biological data confirmed that leiodermatolide **(1)** causes spindle dysfunction and mitotic arrest, yet does not bind to purified tubulin directly in a cell-free assay; its mode of action is therefore distinctly different from that of clinically approved tubulin-binding drugs and makes the derivatives of leiodermatolide **(1)** as provided here to be most promising candidates for chemotherapy by way of antibody-drug-conjugates

Collectively, these data allow the inventors to conclude that the carbamate present at the C.9 position of the natural product leiodermatolide **(1)** is a permissive site for chemical variation, even though an intact hydrogen bond

between the C.7-OH and an adjacent carbonyl at C.9 helps in optimizing the potency. The carbamate site of leiodermatolide **(1)** at C.9 is therefore identified as the preferred site for the attachment of a clinically viable linker that connects leiodermatolide **(1)** or a derivative thereof to an appropriate monoclonal antibody to give a clinically viable ADC.

Alternatively, the C.7 hydroxyl group of leiodermatolide **(1)** can serve as site for the attachment of a clinically viable linker that connects to an appropriate monoclonal antibody to give a clinically viable ADC.

These data also show that the bond that connects a clinically viable linker to C.7 or C.9 on the framework of leiodermatolide **(1)** is important for optimizing the potency. Said attachment can materialize in the form of an ester, ether, acetal, carbamate, carbonate, or thiocarbonate. Preferred functional groups are those which contain a carbonyl subunit, as present in an ester, carbamate, carbonate or thiocarbonate.

Finally, the data show that the δ-lactone group of leiodermatolide **(1)** is less adequate as a linker site for the formation of antibody-drug-conjugates comprising **1** as the cytotoxic payload.

The invention is further illustrated by the following Experimental Part.

Experimental Part

Preparation and Characterization Data of Leiodermatolide (**1**) and Analogues:

**Compound 41. A** solution of trichloroacetyl isocyanate (1.0 M in $CH_2Cl_2$, 16.4 μL,

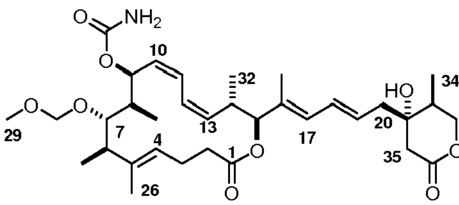

30

16.4 μmol) was added to a precooled solution (-78 °C) of the allylic alcohol 40 (9.0 mg, 15.0 μmol) in $CH_2Cl_2$ (500 μL). The mixture was stirred at -78 °C for 2 h, before being quenched with MeOH (100 μL) at this temperature. After warming and concentration *in vacuo,* the residue was dissolved in $CH_2Cl_2$ (1 mL) and the solution soaked on basic alumina. After 1.5 h, the alumina was loaded onto a short pad of Celite, which was eluted with EtOAc/EtOH (9:1, 12 mL) and the solvent was concentrated *in vacuo.* The residue was purified by flash chromatography (Florisil, hexanes/EtOAc, 1:1 → 1:2) to yield the title compound as a white foam (8.1 mg, 84%). $[\alpha]_{20}^{D}$ = -66.4 (c = 0.94, $CD_2Cl_2$); $^1$H NMR (600 MHz, $CD_2Cl_2$): confirmed; $^{13}$C NMR (150 MHz, $CD_2Cl_2$): confirmed; IR (film): 3452, 3365, 2965, 2931, 1723, 1602, 1455, 1376, 1312, 1259, 1209, 1146, 1092, 1058, 1033, 954, 916, 863, 801, 748, 710, 679 $cm^{-1}$; HRMS (ESIpos): *m*/*z*: calcd. for $C_{36}H_{55}NO_9Na$ [$M^+$+Na]: 668.37800, found 668.37740.

**Leiodermatolide (1).** A solution of compound **41** (9.0 mg, 13.9 μmol) in $CH_2Cl_2$

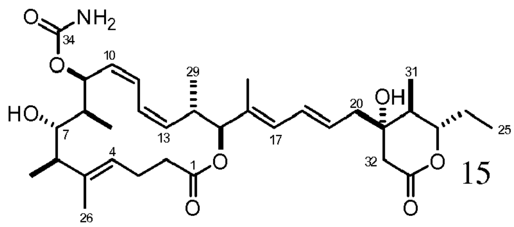

15

(1.6 mL) was cooled to -90 °C ($Et_2O/CO_2/N_2$ cooling bath) before a solution of freshly prepared $Me_2BBr$ (0.5 M in $CH_2Cl_2$, 30.6 μL, 15.3 μmol) was carefully added via the cold wall of the flask. The reaction mixture was allowed to reach -78 °C and was stirred at this temperature for 1.5 h, when a second aliquot of $Me_2BBr$ (0.5 M, 30.6 μL, 15.3 μmol) was introduced. After additional 1.5 h, the mixture was transferred via canula into a vigorously stirred mixture of sat. $NaHCO_3/H_2O$/THF (1:1:1, 10 mL) and the flask was rinsed with THF (2 x 0.7 mL). After stirring for 10 min, the mixture was diluted with EtOAc (10 mL), the aqueous layer was extracted with EtOAc (3 x 10 mL), the combined organic layers were dried over $Na_2SO_4$ and concentrated *in vacuo.* The residue was purified by preparative thin layer chromatography (TLC Silica gel 60 F254 (20 x 20 cm), hexanes/EtOAc, 2:5) to yield the title compound as a white solid (5.1 mg, 61%).

$[\alpha]_{24}^{D}$ = -74.3 (c = 0.41, MeOH); IR (film): 3360, 2963, 2924, 1708, 1605, 1455, 1375, 1312, 1246, 1207, 1148, 1082, 1056, 1040, 986, 949, 915, 778, 745 $cm^{-1}$; MS (ESI) *m/z* (%): 624.4 (100); HRMS (ESIpos): *m/z:* calcd. for $C_{34}H_{51}NO_8Na$ [$M^+$+Na]: 624.35069, found 624.35132.

**Analogue 43.** A solution of allylic carbamate **A4** (1.5 mg, 2.60 µmol) in $CH_2Cl_2$

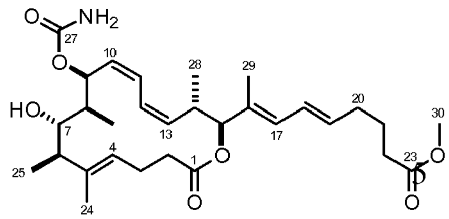

(260 µL) was cooled to -90 °C ($Et_2O/CO_2/N_2$ cooling bath) before a solution of freshly prepared $Me_2BBr$ (0.5 M in $CH_2Cl_2$, 6.4 µL, 3.20 µmol) was carefully added via the cold wall of the flask. The reaction mixture was allowed to reach -78 °C and was stirred at this temperature for 1 h, when a second aliquot of $Me_2BBr$ (0.5 M in $CH_2Cl_2$, 6.4 µL, 3.20 µmol) was introduced. After additional 0.5 h, the mixture was transferred via canula into a vigorously stirred mixture of sat. $NaHCO_3/H_2O$/THF (1:1:1, 3 mL) and the flask was rinsed with THF (2 x 200 µL). After stirring for 0.5 h, the mixture was diluted with EtOAc (3 mL), the aqueous layer was extracted with EtOAc (3 x 3 mL), the combined organic layers were dried over $Na_2SO_4$ and concentrated *in vacuo.* The residue was purified by preparative thin layer chromatography (TLC Silica gel 60 F254 (20 x 20 cm), MeOH/$CH_2Cl_2$, 7:93) to yield the title compound as a colorless viscous liquid (1.0 mg, 67%, ~90% purity). $^1H$ NMR (600 MHz, $CD_2Cl_2$): confirmed; $^{13}C$ NMR (150 MHz, $CD_2Cl_2$): confirmed; IR (film): 3356, 2952, 2923, 2854, 1731, 1666, 1607, 1456, 1435, 1375, 1316, 1259, 1203, 1149, 1082, 1056, 1026, 988, 965, 892, 797, 745, 699 $cm^{-1}$; HRMS (ESIpos): m/z: calcd. for $C_{30}H_{45}NNaO_7$ [$M^+$+Na]: 554.30882, found 554.30886.

**Compound 44.** A solution of allylic carbamate **B4** (1.4 mg, 2.38 µmol) in $CH_2Cl_2$

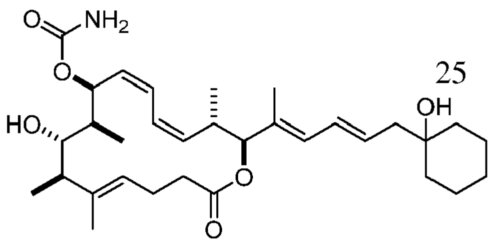

(250 µL) was cooled to -90 °C ($Et_2O/CO_2/N_2$ cooling bath) before a solution of freshly prepared $Me_2BBr$ (0.1 M in $CH_2Cl_2$, 28.6 µL, 2.86 µmol) was carefully added via the cold wall of the flask. The reaction mixture was allowed to reach -78 °C and was stirred at this temperature for 0.5 h, when a second aliquot of $Me_2BBr$ (0.1 M in $CH_2Cl_2$, 28.6 µL, 2.86 µmol) was introduced. After additional 0.5 h, the mixture was transferred via canula into a vigorously stirred mixture of sat. $NaHCO_3/H_2O$/THF (1:1:1, 3 mL) and the flask was rinsed with THF (2 x 200 µL). After stirring for 0.5 h, the mixture was diluted with EtOAc (3 mL), the aqueous layer was extracted with EtOAc (3 x 3 mL), the combined organic layers were dried over $Na_2SO_4$ and concentrated *in vacuo.* The residue was purified by preparative thin layer chromatography (TLC Silica gel 60 F254 (20 x 20 cm), MeOH/$CH_2Cl_2$, 7:93) to yield the title compound as a colorless viscous liquid (0.84 mg, 65%). $^1H$ NMR (600 MHz, $CD_2Cl_2$): δ = 6.53 (dd, *J* = 11.5, 11.4 Hz, 1 H), 6.38 (dd, *J* = 11.6, 11.0 Hz, 1 H), 6.31 (ddt, *J* = 15.0, 10.9, 1.2 Hz, 1 H), 6.08 (dd, *J* = 10.9, 1.1 Hz, 1 H), 5.90 (d, *J* = 10.1 Hz, 1 H), 5.81 (dt, *J* = 15.3, 7.7 Hz, 1 H), 5.53 (t, *J* = 10.4 Hz, 1 H), 5.39 - 5.33 (m, 1 H), 5.12 - 5.08 (m, 1 H), 5.06 (d, *J* = 10.3 Hz, 1 H), 4.64 (br s, 2H), 3.26 (br t, *J* = 9.5 Hz, 1 H), 3.03 - 2.93 (m, 1H), 2.46 (dq, *J* = 13.2, 6.6 Hz, 1 H), 2.32 - 2.27 (m, 1 H), 2.27 - 2.24 (m, 2H), 2.24 - 2.10 (m, 3H), 2.04 - 1.96 (m, 2H), 1.78 (d, *J* = 1.1 Hz, 3H), 1.74 (dd, *J* = 14.6, 7.3 Hz, 1 H), 1.62 - 1.54 (m, 3H), 1.51 - 1.44 (m, 3H), 1.42 (s, 3H), 1.41 - 1.38 (m, 2H), 1.36 - 1.30 (m, 2H), 1.13 (d, *J* = 6.7 Hz, 3H), 1.09 (d, *J* = 7.3 Hz, 3H), 0.87 (d, *J* = 6.7 Hz, 3H); HRMS (ESIpos): m/z: calcd. for $C_{32}H_{49}NO_6Na$ [$M^+$+Na]: 566.34521, found 566.34571.

**Analogue 42.** A solution of allylic carbamate **B4** (0.8 mg, 1.23 µmol) in $CH_2Cl_2$

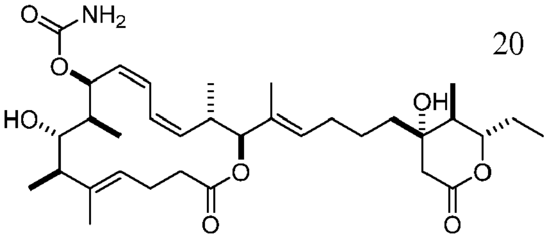

(120 $\mu$L) was cooled to -90 °C ($Et_2O/CO_2/N_2$ cooling bath) before a solution of freshly prepared $Me_2BBr$ (0.1M in $CH_2Cl_2$, 14.9 $\mu$L, 1.49 $\mu$mol) was carefully added via the cold wall of the flask. The reaction mixture was allowed to reach -78 °C and was stirred at this temperature for 1 h, when a second aliquot of $Me_2BBr$ (0.1 M in $CH_2Cl_2$, 14.9 $\mu$L, 1.49 $\mu$mol) was introduced. After additional 0.5 h, the mixture was transferred via canula into a vigorously stirred mixture of sat. $NaHCO_3/H_2O$/THF (1:1:1, 3 mL) and the flask was rinsed with THF (2 x 200 $\mu$L). After stirring for 0.5 h, the mixture was diluted with EtOAc (3 mL), the aqueous layer was extracted with EtOAc (3 x 3 mL), the combined organic layers were dried over $Na_2SO_4$ and concentrated *in vacuo.* The residue was purified by preparative thin layer chromatography (TLC Silica gel 60 F254 (20 x 20 cm), $MeOH/CH_2Cl_2$, 7:93) to yield the title compound as a colorless viscous liquid (0.56 mg, 63%, ~90% purity). $^1H$ NMR (500 MHz, $CD_2Cl_2$): $\delta$ = 6.52 (dd, *J* = 11.5 , 11.4 Hz, 1 H), 6.37 (dd, *J* = 11.3, 11.2 Hz, 1 H), 5.90 (d, *J* = 10.1 Hz, 1 H), 5.57 - 5.45 (m, 3H), 5.13 - 5.06 (m, 1 H), 5.03 (d, *J* = 10.3 Hz, 1 H), 4.64 (br s, 2H), 3.87 (ddd, *J* = 10.3, 7.6, 3.2 Hz, 1 H), 3.26 (br t, *J* = 9.7 Hz, 1 H), 3.00 - 2.90 (m, 1 H), 2.74 (d, *J* = 16.5 Hz, 1 H), 2.45 (dq, *J* = 10.5, 6.6 Hz, 1 H), 2.34 (d, *J* = 16.5 Hz, 1 H), 2.32 - 2.26 (m, 1 H), 2.24 - 2.16 (m, 2H), 2.17 - 2.11 (m, 1 H), 2.11 - 2.03 (m, 2H), 2.03 - 1.95 (m, 1 H), 1.88 - 1.78 (m, 2H), 1.77 - 1.70 (m, 1 H), 1.65 (d, *J* = 1.3 Hz, 3H), 1.64 - 1.55 (m, 2H), 1.49 - 1.43 (m, 2H), 1.42 (d, *J* = 1.4 Hz, 3H), 1.40 - 1.33 (m, 2H), 1.13 (d, *J* = 6.7 Hz, 3H), 1.08 (d, *J* = 7.3 Hz, 3H), 1.01 (t, *J* = 7.4 Hz, 3H), 0.97 (d, *J* = 6.9 Hz, 3H), 0.86 (d, *J* = 6.6 Hz, 3H); HRMS (ESIpos): m/z: calcd. for $C_{34}H_{53}NO_8Na$ [$M^+$+Na]: 626.36634, found 626.36684.

**Analogue 45.** A solution of allylic acetate **F4** (4.8 mg, 7.4 $\mu$mol) in $CH_2Cl_2$ (0.8 mL)

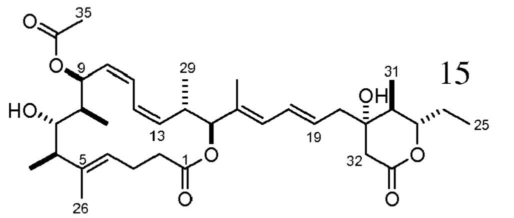

was cooled to -90 °C ($Et_2O/CO_2/N_2$ cooling bath) before a solution of freshly prepared $Me_2BBr$ (0.5 M in $CH_2Cl_2$, 16.4 $\mu$L, 8.2 $\mu$mol) was carefully added via the cold wall of the flask. The mixture was allowed to reach - 78 °C and was stirred at this temperature for 1.5 h, when a second aliquot of $Me_2BBr$ (0.5 M, 16.4 $\mu$L, 8.2 $\mu$mol) was introduced. The reaction mixture was stirred for 1.5 h, when a third aliquot of $Me_2BBr$ (0.5 M, 16.4 $\mu$L, 8.2 $\mu$mol) was introduced. After additional 1.5 h, the mixture was transferred via canula into a vigorously stirred mixture of sat. $NaHCO_3/H_2O$/THF (1:1:1, 7 mL) and the flask was rinsed with THF (2 x 0.5 mL). After stirring for 10 min, the mixture was diluted with EtOAc (10 mL), the aqueous layer was extracted with EtOAc (3 x 10 mL), the combined organic layers were dried over $Na_2SO_4$ and concentrated *in vacuo.* The residue was purified by preparative thin layer chromatography (TLC Silica gel 60 F254 (20 x 20 cm), hexanes/EtOAc, 1:1) to yield the title compound as an off-white solid (2.4 mg, 54% yield). $^1H$ NMR (600 MHz, $CD_2Cl_2$): confirmed; $^{13}C$ NMR (150 MHz, $CD_2Cl_2$): confirmed; IR (film): 3472, 2956, 2924, 2854, 1733, 1459, 1371, 1259, 1246, 1207, 1149, 1100, 1015, 979 $cm^{-1}$MS (ESI) m/z (%): 593.4 (100); HRMS (ESIpos): *m/z:* calcd. for $C_{34}H_{50}O_7Na$ [$M^+$+Na]: 593.34487, found 593.34547.

**Compound 46.** A solution of alcohol **40** (4.0 mg, 6.66 $\mu$mol) in $CH_2Cl_2$ (0.71 mL)

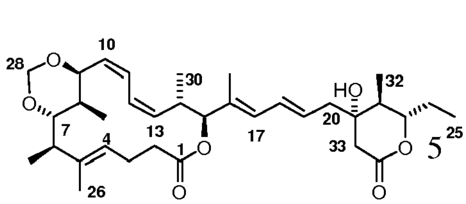

was cooled to -90 °C ($Et_2O/CO_2/N_2$ cooling bath) before a solution of freshly prepared $Me_2BBr$ (0.5 M in $CH_2Cl_2$, 30.6 $\mu$L, 15.3 $\mu$mol) was carefully added via the cold wall of the flask. The mixture was allowed to reach -78 °C and was

stirred at this temperature for 1.5 h, when a second aliquot of $Me_2BBr$ (0.5 M, 14.6 μL, 15.3 μmol) was introduced. This was repeated after another 1.5 h. After additional 1.5 h, the mixture was transferred via canula into a vigorously stirred mixture of sat. $NaHCO_3/H_2O$/THF (1:1:1, 7 mL) and the flask was rinsed with THF (2 x 0.5 mL). After stirring for 10 min, the mixture was diluted with EtOAc (10 mL), the aqueous layer was extracted with EtOAc (3 x 10 mL), the combined extracts were dried over $Na_2SO_4$ and concentrated. The residue was purified by preparative thin layer chromatography (TLC Silica gel 60 F254 (20 x 20 cm), hexanes/EtOAc, 3:2) to give the title compound as an off-white solid (1.1 mg, ~75% purity, 20% yield). $^1H$ NMR (600 MHz, $CD_2Cl_2$): δ = confirmed; $^{13}C$ NMR (150 MHz, $CD_2Cl_2$): δ = confirmed; IR (film): 3469, 2963, 2924, 2855, 1732, 1458, 1376, 1249, 1206, 1146, 1080, 1042, 1006, 898, 800, 739 $cm^{-1}$. MS (ESI) *m/z* (%): 593.4 (100); HRMS (ESIpos): m/z: calcd. for $C_{34}H_{50}O_7Na$ [$M^+$+Na]: 593.34487, found 593.34547.

As shown above, the inventors have modified the substituents on and/or the substructures within the framework of leiodermatolide **(1)** and prepared a set of non-natural analogues. The cytotoxicity of these derivatives and analogues was compared to the cytotoxicity of leiodermatolide **(1)** itself. The analysis of these data showed that the C.7 and C.9 sites and substructures of the framework of this natural product are chemically viable for modification without losing or unduly compromising the biological activity of the natural lead compound. In this way, the inventors proved that leiodermatolide **(1)** qualifies as cytotoxic payload for antibody-drug-conjugates (ADCs), thus showing the preferred linker sites which can be used for the preparation of antibody-drug-conjugates comprising leiodermatolide **(1)** or a derivative thereof as the cytotoxic payload.

## Claims

1. Leiodermatolide derivative of the formula (I)

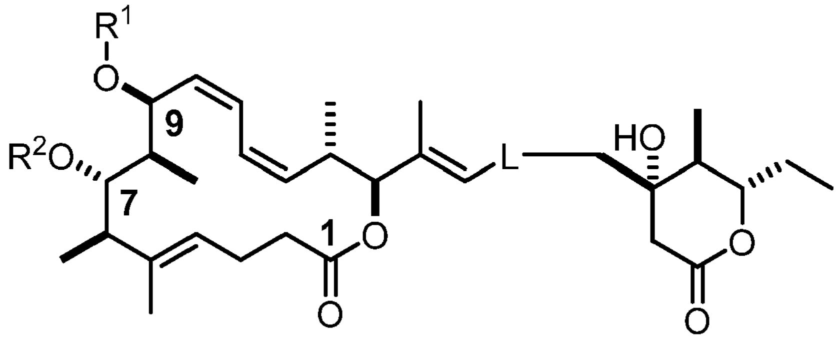

Formula (I)

wherein:

$R^1$ is -CO-$NR_2$, wherein R is the same or different and is hydrogen or a optionally substituted $C_1$ to $C_{12}$-hydrocarbon group, preferably alkyl group, or
$R^1$ is a linking group, which is capable of linking the leiodermatolide unit to an antibody;
$R^2$ is hydrogen, an optionally substituted $C_1$ to $C_{12}$-hydrocarbon group,
preferably alkyl group, or $R^2$ is a linking group, which is capable of linking the leiodermatolide unit to an antibody;
L represents a trans -CH=CH- group or a -$CH_2$-$CH_2$- group,
with the proviso that

a. only one of $R^1$ and $R^2$ is a linking group, and
b. the compound is excluded wherein $R^1$ is -CO-$NH_2$, $R^2$ is hydrogen and L represents a trans -CH=CH- group.

2. Leiodermatolide derivative of the formula (I) according to claim 1, wherein $R^1$ is a linking group having at least one functional group, preferably terminal to the leiodermatolide unit, said functional group being capable of chemically bonding to an antibody.

3. Leiodermatolide derivative of the formula (I) according to claim 1, wherein $R^2$ is a linking group having at least one

functional group, preferably terminal to the leiodermatolide unit, said functional group being capable of chemically bonding to an antibody.

**4.** Leiodermatolide derivative of the formula (I) according to claim 1 or 2, wherein $R^1$ is a linking group having a hydrocarbon chain comprising one or more alkylene groups, arylene groups, heteroarylene groups or different groups thereof in the chain, which chain may contain one or more heteroelements selected from -O-, -S-, -N- and which chain may be substituted, whereby the hydrocarbon chain is having preferably 1 to 40, preferably 6 to 30 carbon atoms in the chain and is preferably substituted terminally to the leiodermatolide unit with one or more substituents selected from =O, -OH, -SH, -SSR, -COOR, $-C(O)NH_2NH_2$, $-CO-NR_2$, $-NR_2$, ,-N(C(O)CH=CHC(O))-, $-OC(O)CH_2X$, -X, X being halogen, R being same or different and being hydrogen or a hydrocarbon group having 1 to 20, preferably 1 to 6 carbon atoms.

**5.** Leiodermatolide derivative of the formula (I) according to claim 1 or 3, wherein $R^2$ is a linking group having a hydrocarbon chain comprising one or more alkylene groups, arylene groups, heteroarylene groups or different groups thereof in the chain, which chain may contain one or more heteroelements selected from -O-, -S-, -N- and which chain may be substituted, whereby the hydrocarbon chain is having preferably 1 to 40, preferably 6 to 30 carbon atoms in the chain and is preferably substituted terminally to the leiodermatolide unit with one or more substituents selected from =O, -OH, -SH, -SSR, -COOR, $-C(O)NH_2NH_2$, $-CO-NR_2$, $-NR_2$, ,-N(C(O)CH=CHC(O))-, $-OC(O)CH_2X$, -X, X being halogen, R being same or different and being hydrogen or a hydrocarbon group having 1 to 20, preferably 1 to 6 carbon atoms.

**6.** Leiodermatolide derivative of the formula (I) as defined in claim 1, wherein
$R^1$ is $-(CO)_n-(NH/O/S)_m-Rw$ wherein Rw is a linear or branched polyoxyalkylene hydrocarbon group or a linear or a branched polyalkylene hydrocarbon group, each hydrocarbon group having 1 to 20, preferably 1 to 12 carbon atoms which may be preferably terminally substituted with one or more substituents selected from =O, -OH, -SH, -SSR, -COOR, - $C(O)NH_2NH_2$, $-CO-NR_2$, $-NR_2$, , -N(C(O)CH=CHC(O))-, $-OC(O)CH_2X$, -X, X being halogen, R being same or different and being hydrogen or a hydrocarbon group having 1 to 20, preferably 1 to 6 carbon atoms;
n = 0 or 1; m = 0 or 1 with m = 0 when n = 0;
$R^2$ is hydrogen or a optionally substituted $C_1$ to $C_6$-alkyl group; and
L represents a trans -CH=CH- or $-CH_2-CH_2-$ group.

**7.** Leiodermatolide derivative of the formula (I) as defined in claim 1, wherein $R^1$ is $-CO-NR_2$, wherein R is the same or different and is hydrogen or a optionally substituted $C_1$ to $C_6$-alkyl group;
$R^2$ is $-(CO)_n-(NH/O)_m-Rw$ wherein Rw is a linear or branched polyoxyalkylene hydrocarbon group or a linear or a branched polyalkylene hydrocarbon group, each hydrocarbon group having 1 to 20, preferably 1 to 12 carbon atoms which may be preferably terminally substituted with one or more substituents selected from from =O, -OH, -SH, -SSR, -COOR, - $C(O)NH_2NH_2$, $-CO-NR_2$, $-NR_2$, , -N(C(O)CH=CHC(O))-, $-OC(O)CH_2X$, -X, X being halogen, R being same or different and being hydrogen or a hydrocarbon group having 1 to 20, preferably 1 to 6 carbon atoms; n = 0 or 1; m = 0 or 1 with m = 0 when n = 0, and
L represents a trans -CH=CH- or $-CH_2-CH_2-$ group.

**8.** Leiodermatolide derivative of the formula (I) as defined in claim 1 wherein:

$R^1$ is $-CO-NR_2$, wherein R is the same or different and is hydrogen or a optionally substituted $C_1$ to $C_6$-alkyl group;
$R^2$ is hydrogen or an optionally substituted $C_1$ to $C_6$-alkyl group;
L represents a $-CH_2-CH_2-$ group.

**9.** Use of a Leidodermatolide derivative of the formula (I) according to any of claims 1 to 8 for preparing a pharmaceutical agent or drug compound for use in medicine.

**10.** Use according to claim 9 wherein the pharmaceutical is an antibody-drug-conjugate.

**11.** Antibody-drug-conjugate comprising a Leidodermatolide derivative of the formula (I) according to any of claims 1 to 8.

Figur 1

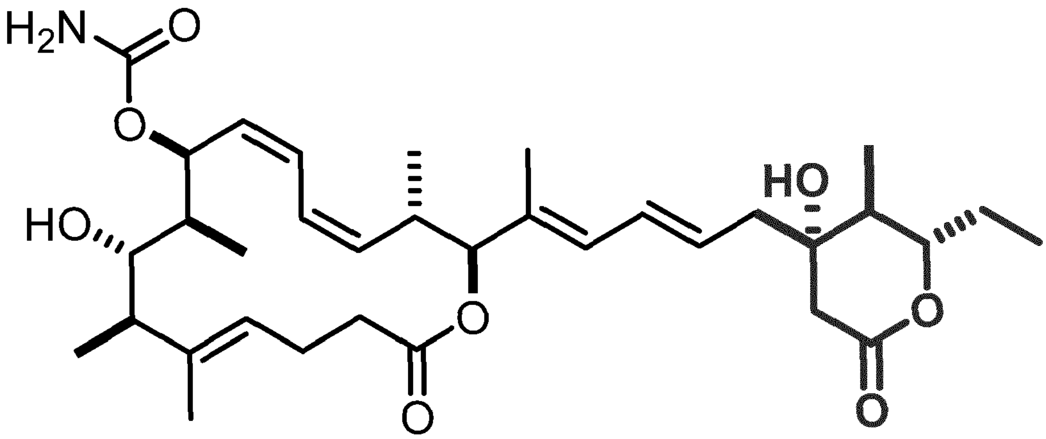

Leiodermatolide (**1**)

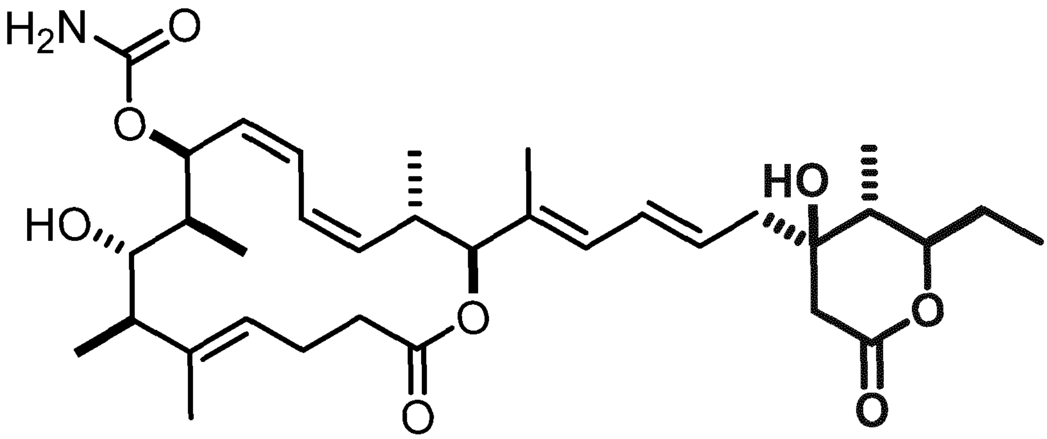

Leiodermatolide (2)

Figur 2

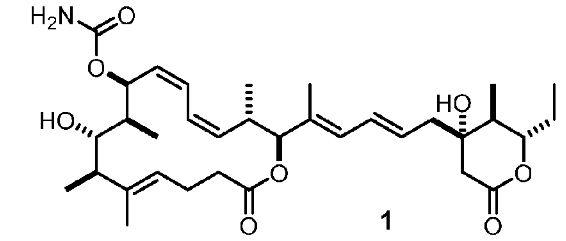

1

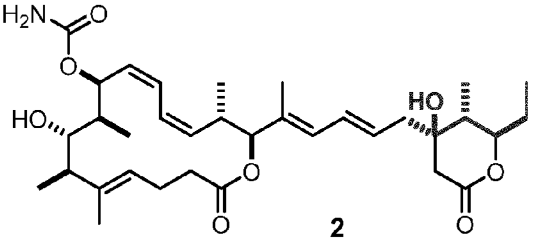

2

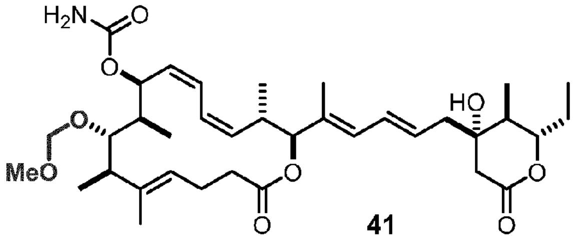

41

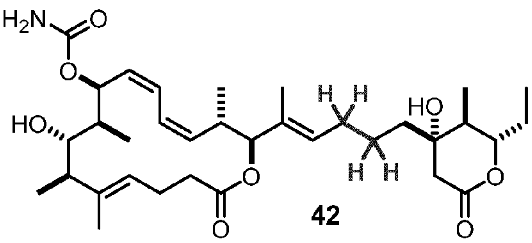

42

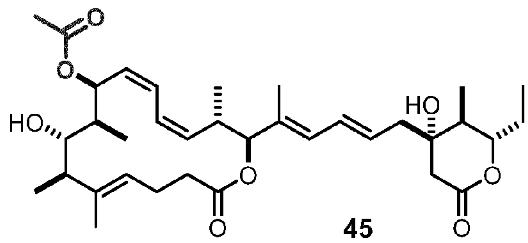

45

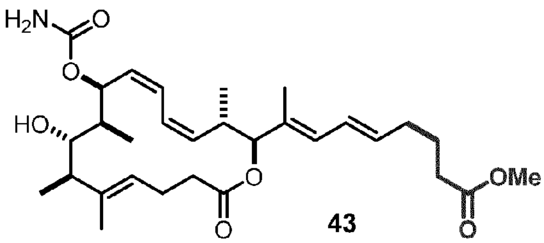

43

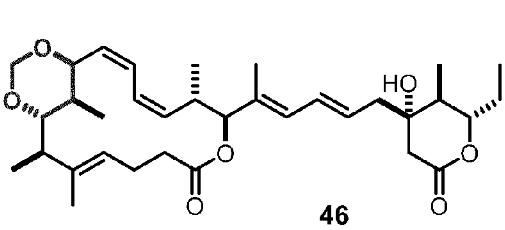

46

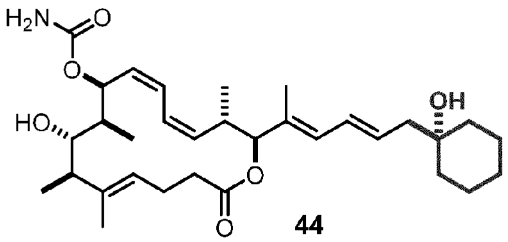

44

Figure 3

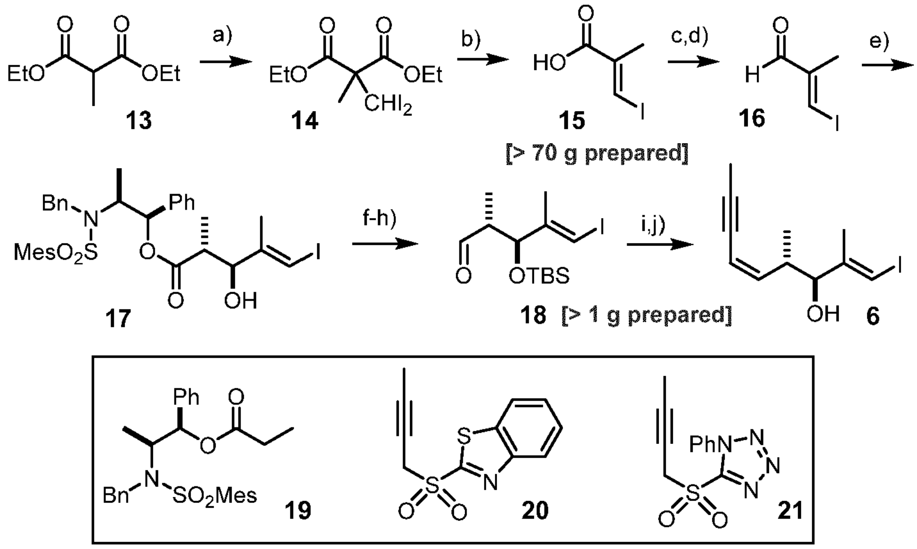


13, 14, 15 [> 70 g prepared], 16, 17, 18 [> 1 g prepared], 6, 19, 20, 21

Figure 4

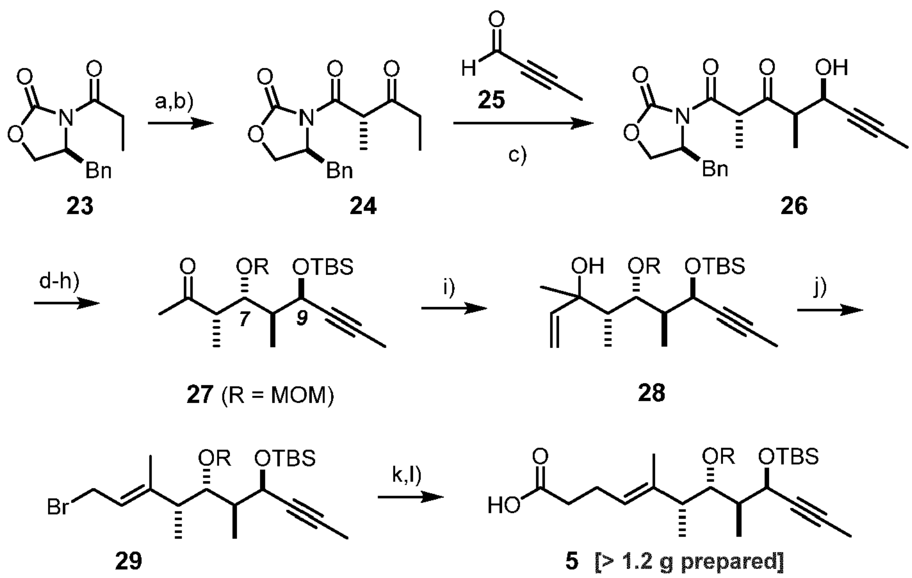


23, 24, 25, 26, 27 (R = MOM), 28, 29, 5 [> 1.2 g prepared]

Figure 5

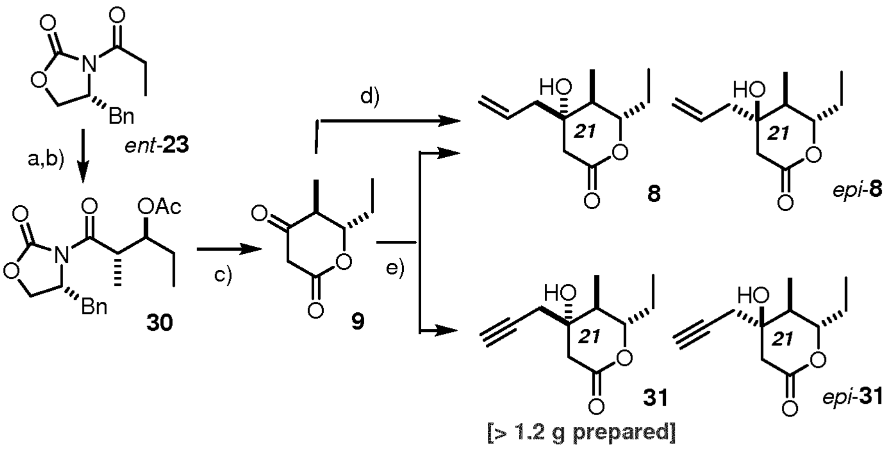

ent-**23**, **30**, **9**, **8**, epi-**8**, **31**, epi-**31**

[> 1.2 g prepared]

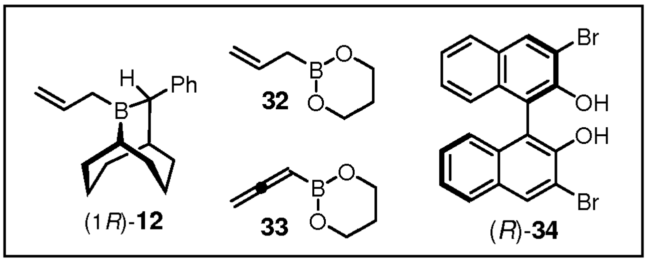
(1*R*)-**12**, **32**, **33**, (*R*)-**34**

Figure 6

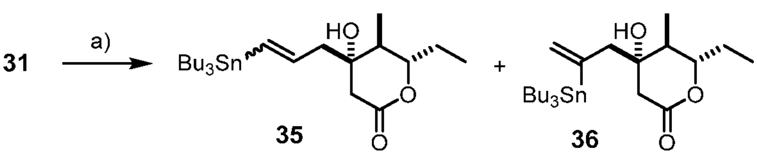

**31**, **35**, **36**

Figure 7

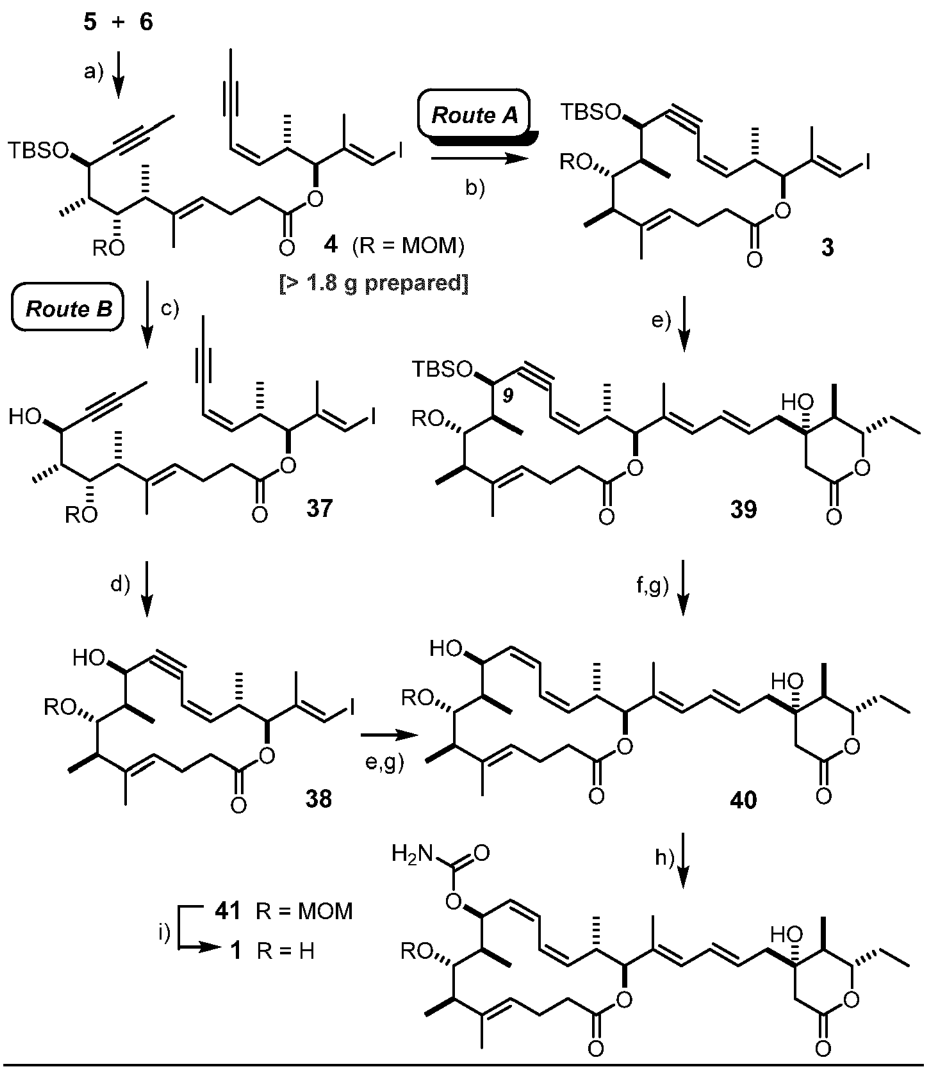
5 + 6
a)
4 (R = MOM)
[> 1.8 g prepared]
Route A
b)
3
Route B
c)
37
e)
39
9
d)
f,g)
38
e,g)
40
h)
41 R = MOM
i)
1 R = H

Figure 8

- **Analogue 43**

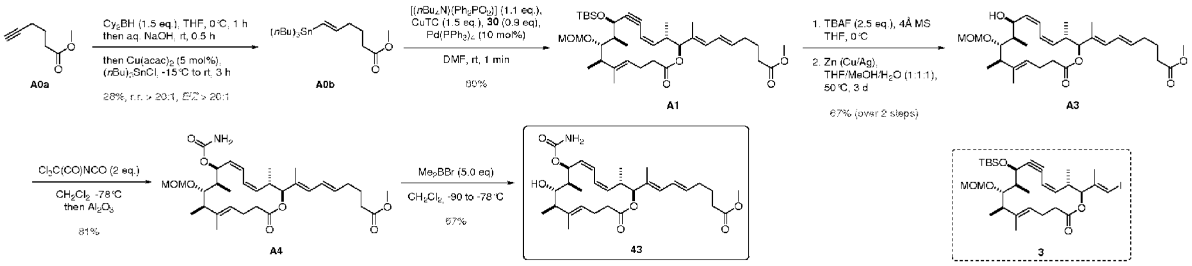


- **Analogue 44**

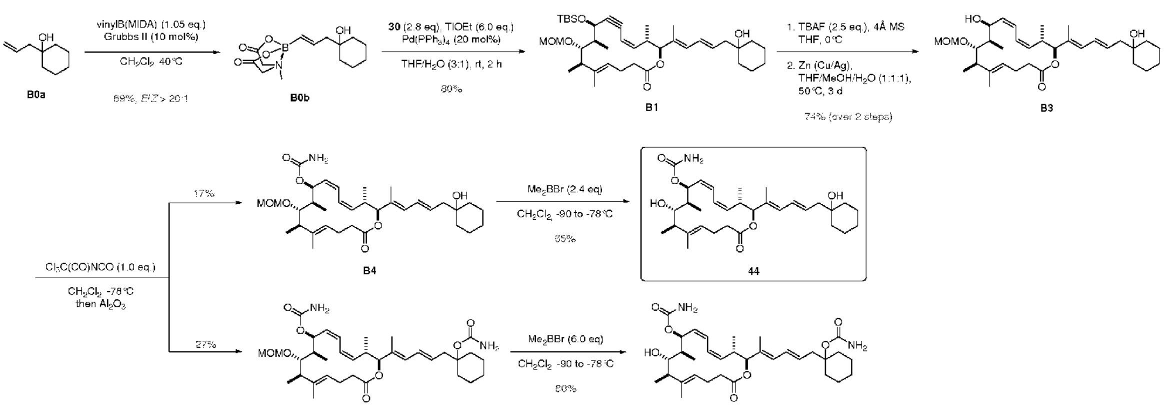

Figure 9

- **Analogue 42**

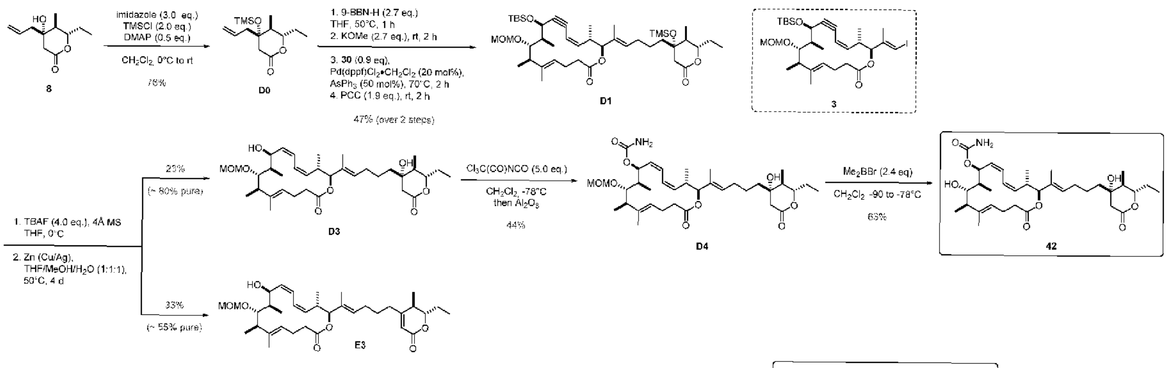


- **Analogues 45 & 46**

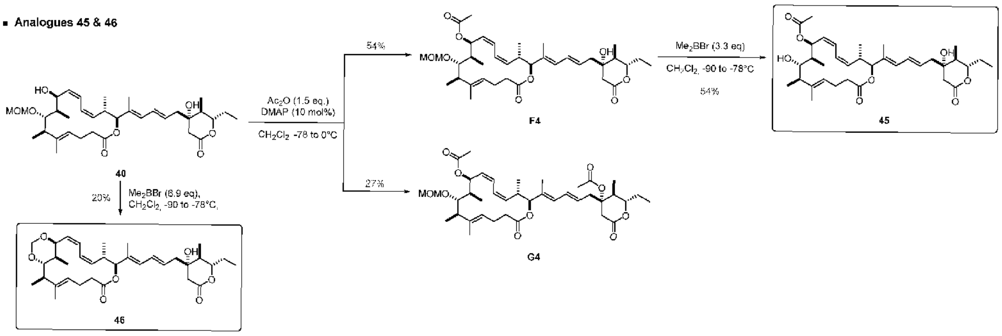

Europäisches Patentamt
European Patent Office
Office européen des brevets

# EUROPEAN SEARCH REPORT

Application Number
EP 14 18 9765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | MAILHOL ET AL.: "Synthesis, Molecular Editing, and Biological Assessment of the Potent Cytotoxin Leiodermatolide", J. AM. CHEM. SOC., vol. 136, no. 44, 27 October 2014 (2014-10-27), pages 15719-15729, XP055167856, ISSN: 0002-7863, DOI: 10.1021/ja508846g<br>* the whole document *<br>----- | 1-11 | INV.<br>C07D407/06<br>A61K31/366<br>A61P35/02 |
| X,D<br><br>Y | WILLWACHER ET AL.: "Divergent Total Synthesis of the Antimitotic Agent Leiodermatolide", ANGEW. CHEM. INT. ED., vol. 51, no. 48, 26 November 2012 (2012-11-26), pages 12041-12046, XP055167831, ISSN: 1433-7851, DOI: 10.1002/anie.201206670<br>* Page 12043, scheme 5: compound 33 = present compound 41; page 12041, paragraph bridging left and right column; page 12044, right column, penultimate paragraph. *<br>----- | 1<br><br>1-11 | TECHNICAL FIELDS SEARCHED (IPC)<br>C07D<br>A61K<br>A61P |
| X,D<br><br>Y | WO 2008/019135 A1 (HARBOR BRANCH OCEANOGRAPHIC [US]; WRIGHT AMY E [US]; REED JOHN K [US];) 14 February 2008 (2008-02-14)<br>* Abstract; page 5, lines 8-20, 27-30; claims. *<br>----- | 1-11<br><br>1-11 | |
| | -/-- | | |

The present search report has been drawn up for all claims

| Place of search | Date of completion of the search | Examiner |
|---|---|---|
| Munich | 9 February 2015 | Weisbrod, Thomas |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 18 9765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | CHARI ET AL.: "Antibody-Drug Conjugates: An Emerging Concept in Cancer Therapy", ANGEW. CHEM. INT. ED., vol. 53, no. 15, 7 April 2014 (2014-04-07), pages 3796-3827, XP055167825, ISSN: 1433-7851, DOI: 10.1002/anie.201307628<br>* The whole document; e.g. section 4.2. *<br>----- | 1-11 | |
| | | | TECHNICAL FIELDS SEARCHED (IPC) |

The present search report has been drawn up for all claims

2

| Place of search | Date of completion of the search | Examiner |
|---|---|---|
| Munich | 9 February 2015 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 18 9765

This annex lists the patent family members relating to the patent documents cited in the above-mentioned European search report. The members are as contained in the European Patent Office EDP file on
The European Patent Office is in no way liable for these particulars which are merely given for the purpose of information.

09-02-2015

| Patent document cited in search report | Publication date | Patent family member(s) | Publication date |
|---|---|---|---|
| WO 2008019135 A1 | 14-02-2008 | US 2008033035 A1<br>WO 2008019135 A1 | 07-02-2008<br>14-02-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8431724 B
- WO 2008019135 A


### Non-patent literature cited in the description


- Anticancer Agents from Natural Products. CRC Press, 2005
- **PATERSON, I. ; DALBY, S. M. ; ROBERTS, J. C. ; NAYLOR, G. J. ; GUZMAN, E. A. ; ISBRUCKER, R. ; PITTS, T. P. ; LINLEY, P. ; DIVLIANSKA, D. ; REED, J. K.** *Angew. Chem. Int. Ed.,* 2011, vol. 50, 3219-3223
- *Angew. Chem. Int. Ed.,* 2012, vol. 51, 12041-46
- *Angew. Chem. Int. Ed.,* 2014, vol. 53, 2692-2995
- **R. V. J. CHARI ; M. L. MILLER ; W. C. WIDDISON.** *Angew. Chem. Int. Ed.,* 2014, vol. 53, 3796